# EUROPEAN PATENT APPLICATION

(11) **EP 1 612 557 A2**
(43) Date of publication of application: **04.01.2006**
(21) Application number: 05018209.6
(22) Date of filing: 20.10.2000
(51) Int. Cl.: G01N 33/543, G01N 33/545, C12Q 1/68

(54) **Binding surface**

(30) Priority: 23.10.1999 GB 9925016
(62) Divisional of application: 00971529.3
(71) Applicant: Plasso Technology Limited, Sheffield S1 4DP (GB)
(72) Inventor: Short, Robert c/o University of Sheffield, Sheffield S1 3JD (GB); Whittle, Jason c/o University of Sheffield, Sheffield S1 3JD (GB)
(74) Representative: Docherty, Robert Charles

(57) **Abstract**

The invention relates to the use of plasma polymerisation of organic molecule to treat surfaces to which biological molecules may bind and be assayed.

## Description

The invention relates to plasma polymerisation of organic molecules and the use of the plasma so formed in the treatment of assay surfaces to which biological molecules can bind.

Currently the use of solid phase assay systems has greatly facilitated the processing and/or analysis of multiple biological samples. This has become a highly automated methodology. Typically, solid phase assays comprise either the immobilisation of the agent to be assayed on a solid, or at least semi-solid, surface or the immobilisation of agents used to assay a biological agent.

The results derived from such assays have greatly assisted clinicians in their diagnosis of various human disorders. They have also enabled environmental authorities to monitor the presence of environmental pollutants and the presence of various infectious agents that may be present in our environment and/or food. Assays of this type are often laborious and time consuming. It is important that assays are sensitive and reliable.

For example, and not by way of limitation, immunoassays have utility in monitoring the presence of various biological molecules. Immunoassays, using solid phase technology, typically involve the immobilisation of either an antibody, or antibody fragment, on the solid surface. Alternatively, an antigen is immobilised. The antibody, or antigen, binds the solid phase surface such that the biologically active moieties are not occluded. The amount of bound antibody, or antigen, is monitored by a variety of methods which are well known in the art.

For example, and not by way of limitation, an antigen which is immobilised is incubated with an antibody to the antigen. Typically the antibody is modified by the linking of either an enzyme (eg horse radish peroxidase, alkaline phosphatase), a fluorescent label ( eg fluorescein, rhodamine) or radioisotope ( eg ³⁵S, ³²P ) which is readily detectable. This can be done either by providing the enzyme with suitable substrate(s) or exciting the fluorescent label with an appropriate wavelength of light.

Alternatively the bound complex of antibody and antigen can be monitored by use of a secondary antibody specific for the bound antibody which is labelled as detailed above.

Alternatively the antibody may be immobilised followed by binding of the antigen to the immobilised antibody. Detection methods are then used to monitor the complex. This can be either be direct labelling of the antigen or by using a labelled antibody recognising a different epitope to that recognised by the immobilised antibody. Substrates to which proteins can bind are well known in the art and include nitrocellulose and charged nylon membranes. These membranes also have affinity for nucleic acids.

Additionally the use of solid phase assays has been adapted to identify potential drugs by their binding to immobilised target molecules in drug selection screens. These molecules may be receptors involved in signal transduction pathways; protein kinases involved in said signal transduction pathways; or ligands which mediate signals both within a cell and between cells. Alternatively, nucleic acid is immobilised on solid surfaces to identify ligands which bind specifically to either a nucleic conformation or a specific sequence. Antagonists identified by these assays have use in various disorders including cancer, respiratory diseases and inherited genetic diseases. The technician conducting the screen has to conduct many thousands of assays to identify potential candidate agents.

Typically solid phase assays are conducted in assay dishes containing multiple wells which are coated with the molecule of interest. These multi-well application dishes are normally manufactured either from glass or plastics which may have variable affinity for the molecule(s) of interest. Alternatively multi-well dishes can be treated chemically to improve their affinity and/or retention of selected molecules at their surface. It is, of course, highly desirable that the treated surface binds with the target molecule with high affinity and retention but also allows the bound molecule to retain most, if not all, of its biological activity thereby providing a sensitive and reliable assay.

An example of such a treatment regime for solid phase surfaces is described in GB2016687. The patent describes the treatment of binding surfaces with polysaccharides. Surfaces treated in this way show increased affinity for both antibodies and antigens. W08603840 describes solid phase assay surfaces manufactured from specialised resins as an alternative to the use of assay containers manufactured from plastics such as polystyrene. Specifically, W08603840 discloses the use of the fluorinated resin polytetrafluoroethylene. W09819161 describes the coating of solid phase assay surfaces with polyethyleneimine. The treated surfaces show low levels of non-specific adsorption and a high concentration of binding of the target molecule.

We have used plasma polymerisation to treat assay surfaces so that they provide a surface with affinity for biological molecules. Plasma polymerisation is a technique which allows an ultra-thin ( eg ca.200nm) cross linked polymeric film to be deposited on substrates of complex geometry and with controllable chemical functionality. As a consequence, the surface chemistry of materials can be modified, without affecting the bulk properties of the substrate so treated. Plasmas or ionised gases are commonly excited by means of an electric field. They are highly reactive chemical environments comprising ions, electrons, neutrals (radicals, metastables, ground and excited state species) and electromagnetic radiation. At reduced pressure, a regime may be achieved where the temperature of the electrons differs substantially from that of the ions and neutrals. Such plasmas are referred to as "cold" or "non-equilibrium" plasmas. In such an environment many volatile organic compounds ( eg volatile alcohol containing compounds, volatile acid containing compounds, volatile amine containing compounds, or volatile hydrocarbons , neat or with other gases, eg Ar, have been shown to polymerise (H.K. Yasuda, Plasma Polymerisation, Academic Press, London 1985) coating both surfaces in contact with the plasma and those downstream of the discharge. The organic compound is often referred to as the "monomer". The deposit is often referred to as "plasma polymer". The advantages of such a mode of polymerisation potentially include: ultra-thin pin-hole free film deposition; plasma polymers can be deposited onto a wide range of substrates; the process is solvent free and the plasma polymer is free of contamination.

Thin polymeric films can be obtained from the plasmas of volatile organic compounds (at reduced pressure of 10⁻² mbar and ideally less than 100°C). In plasma polymer deposition, there is generally extensive fragmentation of the starting compound or ionised gas and a wide range of the resultant fragments or functional groups are undesirably incorporated into the deposit. By employing a low plasma input power (low plasma power/monomer flow rate ratio) it is possible to fabricate films with a high degree of functional group retention. An example of such a low power/rate ratio is 2W and a flow rate of 2.0sccm. However, other relatively low ratios may be used and are known to those skilled in the art. Alternatively, plasma polymer deposits may be formed by pulsing the plasmas or ionised gases. Plasmas are formed either from single monomer species or in combination with other organic molecules

Co-polymerisation of one or more compounds having functional groups with a hydrocarbon allows a degree of control over surface functional group concentrations in the resultant plasma copolymer (PCP). Suitably, the monomers are ethylenically unsaturated: thus the functional group compound maybe unsaturated carboxylic acid, alcohol or amine, for example, whilst the hydrocarbon is suitably an alkene. PCPs can be deposited directly onto most surfaces, regardless of geometry, making them ideal for treating surfaces such as plastics used in bioassays.

We have exploited plasma polymer deposition to coat suitable substrates for use in, particularly, but not exclusively, immunoassays. We have undertaken plasma polymerisation using allyl alcohol, acrylic acid, octa-1,7-diene and allyl amine onto conventional polystyrene microwells, to form a functionalised surface. We show that surfaces treated with the above identified polymers show selective increased affinity for biological molecules exposed to said surface and allow the assaying of the bound molecule. The surfaces are uniform and enable the reproducible and sensitive assaying of biological molecules bound to the surface.

According to a first aspect of the invention there is provided a surface to which at least one biological molecule is capable of binding characterised in that said surface comprises an area obtainable by plasma polymerisation.

In a preferred embodiment of the invention said surface is part of an assay product.

In a preferred embodiment of the invention said surface is an assay surface for use in the detection of the presence and/or activity of at least one biological molecule bound thereto.

Conventionally assay surfaces embodied by the invention include, by example and not by way of limitation, multiwell microtitre dishes and the like, nitrocellulose or charged nylon membranes. The treatment of membranes with different organic molecules by plasma polymerisation allows the surface functionality of the membrane to be altered so as to provide a membrane to which proteins can selectively bind. Selective western blotting can then be conducted wherein the nitrocellulose or nylon membrane is enriched for a particular protein or family of proteins thereby increasing the sensitivity of the technique. The technique also benefits in so far as proteins from complex mixtures (eg serum) can be selected for thereby enriching the target providing a more sensitive assay.

In yet a further preferred embodiment of the invention said assay is an immunoassay.

In yet a further preferred embodiment of the invention said assay is an enzyme linked assay.

In yet still a further preferred embodiment of the invention said assay is used to identify potential antagonists.

In a further preferred embodiment of the invention said assay is used to identify potential agonists.

Typically, agents which promote a suitable biochemical response are known as agonists and those that prevent, or hinder, a biochemical response are known as antagonists.

In a further preferred embodiment of the invention said biological molecule is a polypeptide or at least the effective part of said polypeptide. Preferably said polypeptide is an antibody, or at least the effective part thereof. Ideally said antibody is a monoclonal antibody, or at least the active Fab fragment thereof.

In a further preferred embodiment of the invention said polypeptide is a receptor.

In yet a further preferred embodiment of the invention said polypeptide is a ligand.

It will be apparent to one skilled in the art that the invention encompasses a means to assay either the presence of a biological molecule or the activity of said molecule. For example and not by way of limitation, a polypeptide receptor is detected by the specific binding of a ligand. Intercellular and/or intracellular signalling via receptor mediated activation of biochemical and/or molecular mechanisms is a fundamental process for regulating cellular and/or tissue homeostasis. The invention, for example, is used to assay the presence of a receptor by monitoring the binding of the cognate labelled ligand.

In a further preferred embodiment of the invention said polypeptide is collagen. Preferably type II collagen.

In a further preferred embodiment of the invention said polypeptide is vitronectin.

In yet a further aspect of the invention there is provided the use of a surface for an assay characterised in that said surface comprises a polymer coating deposited by plasma polymerisation.

Other biological molecules encompassed by the invention include, by example and not by way of limitation, antigens from infectious agents (eg viruses, bacteria, parasites), hormones, tumour rejection antigens, peptides, cytokines, lymphokines, neurotransmitters.

In yet still a further preferred embodiment of the invention said biological molecule is nucleic acid. Preferably said nucleic acid is RNA. Ideally said nucleic acid is DNA.

In a still further preferred embodiment of the invention said surface has been treated by plasma polymerisation with a volatile alcohol.

In a still further preferred embodiment of the invention said surface has been treated by plasma polymerisation with a volatile acid.

In a still further preferred embodiment of the invention said surface has been treated by plasma polymerisation with a volatile amine.

In a still further preferred embodiment of the invention said surface has been treated by plasma polymerisation with a volatile hydrocarbon.

In yet still a further preferred embodiment of the invention said surface has been treated by plasma polymerisation with at least one of allyl alcohol; acrylic acid, octa-1,7-diene; allyl amine. Ideally said plasma polymerisation of said surface is with allyl amine.

Preferably the surface is obtainable by plasma polymerisation of a monomer preparation.

Preferably the monomer preparation consists essentially of an ethylenically unsaturated organic compound.

Preferably the monomer preparation comprises essentially of a single ethylenically unsaturated organic compound.

Preferably the compound is an alkene (eg containing up to 20 carbon atoms and more usually up to 12 carbon atoms, eg 8), a carboxylic acid ( especially α,β - unsaturated carboxylic acid, for example acrylic or methacrylic acid); an alcohol ( especially an α,β - unsaturated alcohol); or an amine ( especially an α,β - unsaturated amine).

Preferably the monomer preparation comprises a mixture of two or more ethylenically unsaturated organic compounds.

Preferably the compounds are selected from the group consisting of: an alkene (eg containing up to 20 carbon atoms and more usually up to 12 carbon atoms, eg 8), a carboxylic acid ( especially α,β - unsaturated carboxylic acid); an alcohol (especially an α,β - unsaturated alcohol); or an amine ( especially an α,β - unsaturated amine).

"Alkene" refers to linear and branched alkenes, of which linear are preferred, containing one or more than one C=C double bond eg an octadiene such as octa-1,7-diene. Dienes form a preferred class of alkenes.

More ideally still said plasma polymer is a co-polymer. Ideally said co-polymer comprises at least one organic monomer with at least one hydrocarbon. Ideally said hydrocarbon is an alkene, eg a diene such as, for example octa 1,7-diene.

The invention also encompasses the use of other compounds to form plasma polymers, for example and not by way of limitation, ethylamine; heptylamine; methacrylic acid; propanol.

According to a second aspect of the invention there is provided a method to treat a surface comprising:
i) providing at least one organic monomer;
ii) creating a plasma of said organic monomer; and
iii) coating the surface with said plasma to provide an assay surface according to the invention.

Preferably said plasma power is created using a plasma power of < 10W and a flow rate of < 5cc/min, under continuous wave conditions. However, in the instance where a pulse wave is used corresponding corrections are made to the plasma power and flow rate as is known by those skilled in the art. It will also be apparent to one skilled in the art that reactor conditions will vary depending on reactor geometry.

According to a further aspect of the invention there is provided a method for performing a biological assay comprising providing a biological molecule bound to a substrate which has the characteristics of a surface which has been treated by plasma polymersation.

An embodiment of the invention will now be described by example only and with reference to the following figures;
Figure 1 is a diagrammatic representation of a plasma polymerisation reactor;
Figure 2 illustrates various plasma polymerisation conditions;
Figure 3a and 3b are graphical representations of the adsorption of IgG to a plasma polymer surfaces after different treatment regimes;
Figure 4 illustrates the adsorption of heat-denatured bovine type II collagen onto various plasma polymer surfaces; and

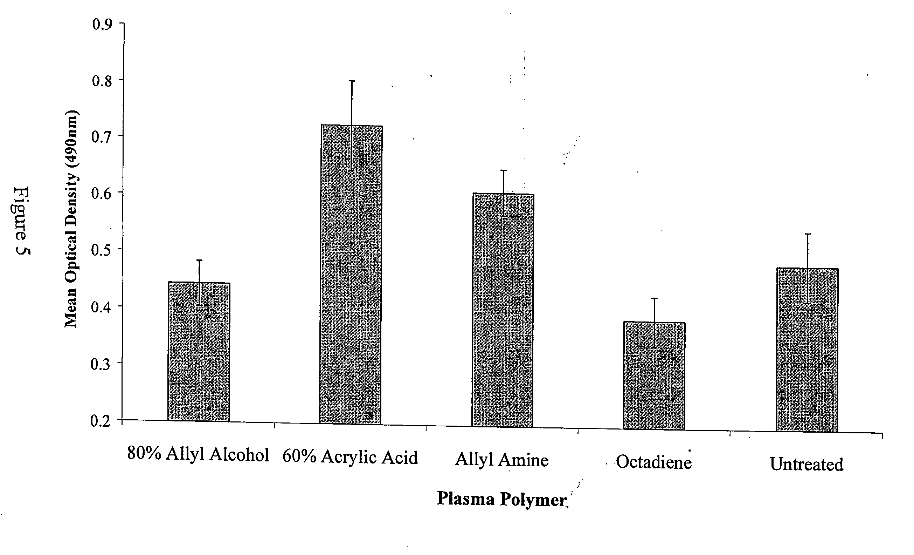

Figure 5 illustrates the adsorption of vitronectin from fetal calf serum onto various plasma polymer surfaces.

### Materials and Methods

Plasma polymerisation is a technique which allows an ultrathin (*ca*. 200nm) crosslinked polymeric film to be deposited on substrates of complex geometry and with controllable chemical functionality. As a consequence, the surface chemistry of materials can be modified, without affecting the bulk properties of the substrate.

### Plasma Polymerisation

Allyl alcohol, acrylic acid, allyl amine and octa-1,7-diene were obtained from Aldrich (UK). They were used as received, save several freeze-thaw cycles to remove dissolved gases prior to use. The substrate for plasma polymerisation was aluminium foil, which was cleaned with acetone and isopropyl alcohol immediately before use.

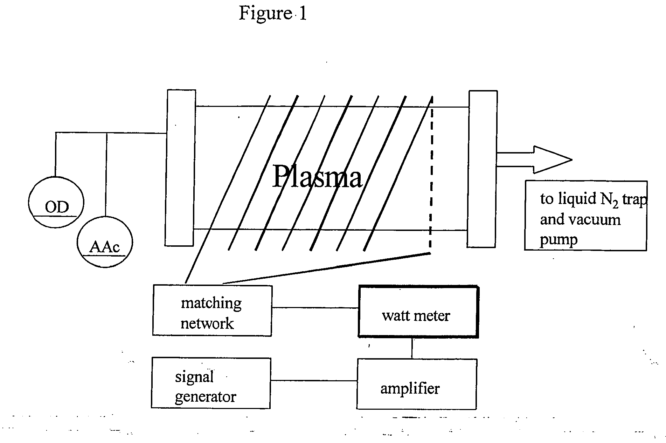

A diagram of the reactor is show in Figure 1. Plasma polymerisation took place in a cylindrical (10 cm internal diameter and 50 cm length) glass reactor capped with two brass flanges. The reactor was connected to a vacuum pump and liquid nitrogen cold trap. Radiofrequency power (13.56MHz) was coupled to the reactor via an impedance matching unit and a copper coil wound externally. The substrates were placed in the 'in-coil' region of the reactor and the vessel was pumped down to a base pressure of 3×10⁻³mbar.

Monomer flow rates were controlled by a needle valve, and estimated by measuring the increase of pressure in the reactor when isolated from the vacuum line. This pressure change is converted to a flow rate using the method described by Yasuda^{[1]} which assumes idea gas behaviour and converts the pressure change into an estimate of monomer flow rate in cm³₍ₛₜₚ₎min⁻¹.

All of the plasma polymers produced for this experiment used a constant monomer flow rate of 1.5cm³₍ₛₜₚ₎min⁻¹ at a pressure of approximately 1.5×10⁻¹mbar and a plasma power of 5W. Depositions took place for a period of 15 minutes, which has been found to be enough time to provide deposits of sufficient thickness to obscure all substrate signals from the XPS spectrum.

### X-Ray Photoelectron Spectroscope

XPS was performed using a VG Clam 2 X-Ray Photoelectron Spectrometer operating in constant analyser energy mode with a dual anode utilising MgKα XRays at a power of 100W. The spectrometer has a base pressure better than 10⁻⁹mbar and an normal operating pressure of better than 10⁻⁸mbar. The instrument is calibrated monthly using a clean gold sample to measure the resolution of the instrument. (At 20eV pass energy the Au 4f_{5/2} peak has a width of *ca*. 1.1eV.) Relative sensitivity factors are measured monthly using a variety of standard polymeric samples. This allows us to quantify the elemental composition of the surface.

A constant take off angle of 30° with respect to the sample surface, was used for all the samples. A survey spectrum was recorded using a pass energy of 100eV to determine the elemental composition of the plasma polymer surface. Core level scans were then taken of all regions of interest (carbon, oxygen and nitrogen core levels where appropriate) at a pass energy of 20eV.

The data was analysed using scienta software, and gaussian-lorenzian component peaks were fitted to the C1s core level spectrum using well established chemical shifts.^{[2]} The hydrocarbon component peak was set at 285eV to correct for any sample charging (typically 4-5eV)

### Enzyme Immunoassay

An enzyme linked immunosorbent assay was performed to estimate the binding of human Immunoglobulin G (IgG) onto the different plasma copolymer surfaces. Utilising the 12-well strips each with different surface treatments, a 96-well plate was assembled with each row containing a different plasma copolymer. A dilution series onto conventional untreated polystyrene microwells was used to determine optimum concentrations for the protein solution. 100µl of IgG diluted to the predetermined concentration (8.8×10-4mg/ml) in phosphate buffered saline (PBS) was added to the first eleven wells on each row. The twelfth well on each row contains PBS only and acts as a background to measure any non-specific binding of the primary and secondary antibodies directly to the surface. The protein was allowed to bind to the surfaces overnight.

After washing the surfaces with PBS-Tween to remove any non-bound material from the wells, a 1% solution of powdered skimmed milk was applied to block any unoccupied binding sites on the surface. After blocking the surfaces for one hour, they were washed again in PBS-Tween and the primary antibody applied. A biotinylated anti-immunoglobulin was used at a concentration of 1 in 2000 and allowed to bind for one hour. The wells were washed again in PBS-Tween to remove any non-bound material, and a solution of avidin conjugated with horseradish peroxidase was applied to the wells at a concentration of 1/1000. After an hour and a further was in PBS-Tween, colour was produced by addition of o-phenylamine diamine dihydrocloride in phosphate-citrate buffer containing 0.03% sodium perborate. When sufficient colour had developed, the reaction was stopped by addition of 2M sulphuric acid.

### Adsorption of type II collagen to plasma polymer surfaces

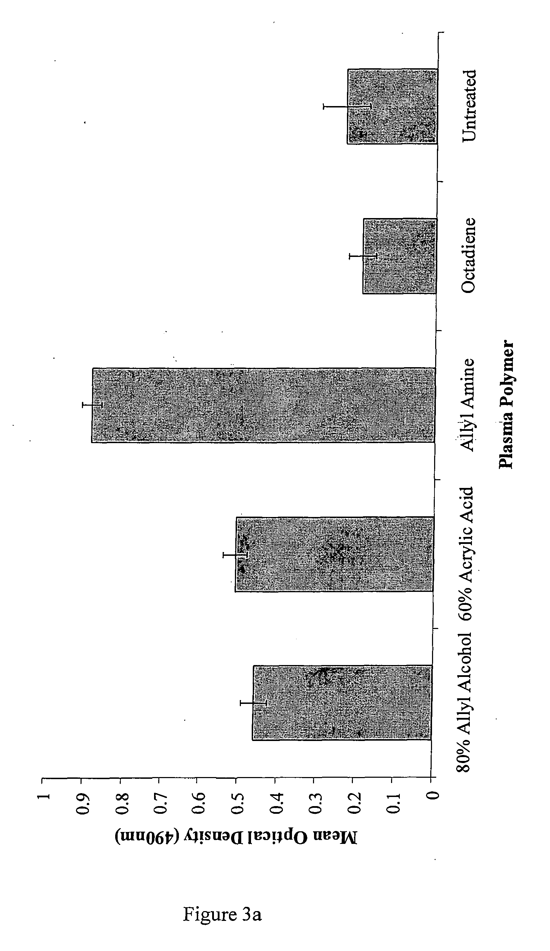

Surfaces were exposed to a 0.0025mg/ml solution of heat-denatured bovine type II collagen. Following blocking with BSA the surfaces were then probed with a monoclonal antibody to the collagen, followed by an alkaline phosphatase-labelled secondary antibody. Substrate was added and the optical density measured at 405nm, see Figure 3. Each column on the graph is the average measurement from 8 wells minus a measured value for non-specific binding. The error bars represent one standard deviation.

### Adsorption of vitronectin from fetal calf serum onto plasma polymer surfaces.

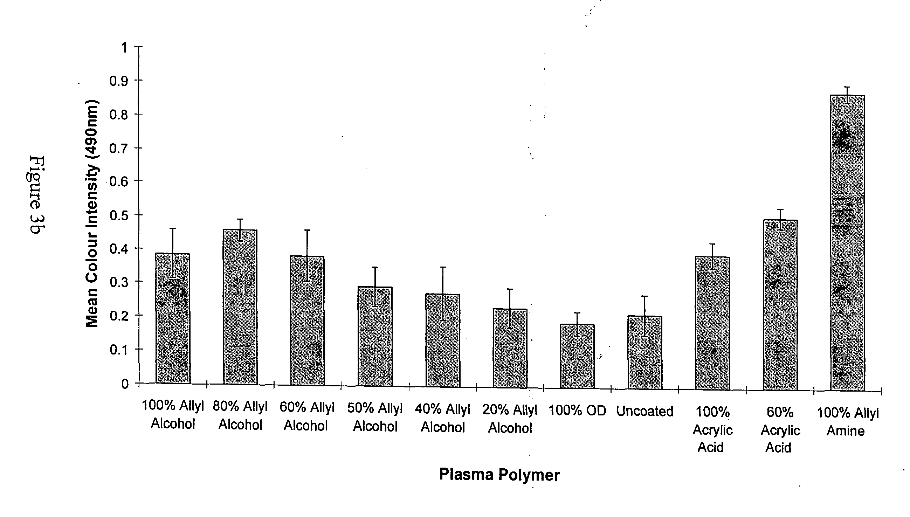

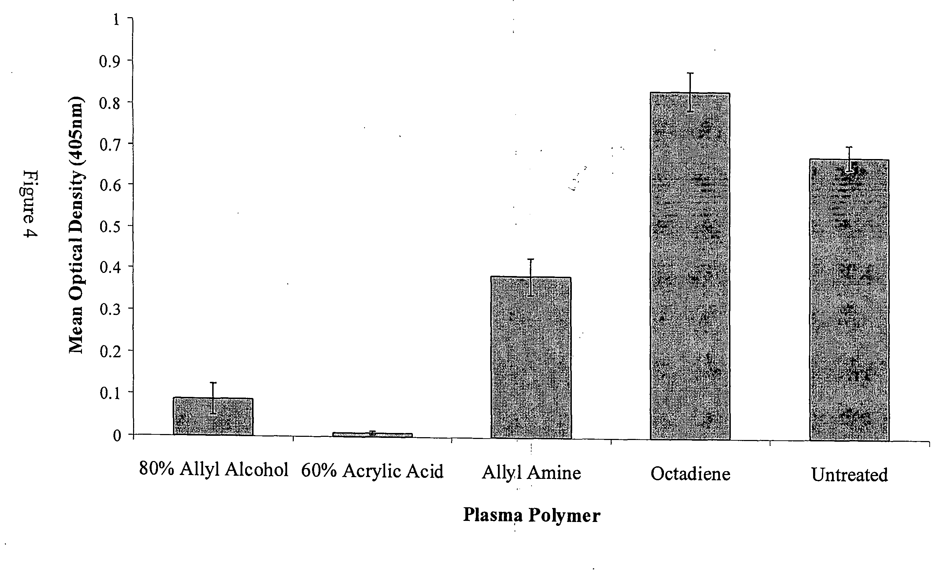

Plasma polymer surfaces were incubated with a solution consisting of fetal calf serum diluted by 1/640 in phosphate buffered saline. Following blocking of the surfaces, they were probed with a polyclonal anti-vitronectin raised in rabbit. A horseradish peroxidase labelled secondary antibody was then applied, and colour produced at 490nm by addition of o-phenylaminediamine substrate, see Figure 4.

### Results

### Surface analysis of plasma copolymers

XPS survey scans of all the plasma polymer surfaces show only the presence of carbon and oxygen (and nitrogen for the allyl amine plasma polymer), illustrating the uniform nature of the films. It is worth noting that plasma polymers deposited from octa-1,7-diene still contain a significant (up to 5%) amount of oxygen. This oxygen is incorporated into the film immediately upon exposure of the films to the atmosphere before they can be analysed by XPS. Also, the presence of H₂O and residual O₂ in the reactor during polymerisation would lead to the formation of oxygen containing groups during plasma treatment. It is reasonable to assume that the similar amount of oxygen also incorporated into the allyl amine plasma polymer is derived from the same source. The widescans were used to determine the surface O/C ratio of the plasma polymers and the C1s core level scans were fitted to obtain functional group information.

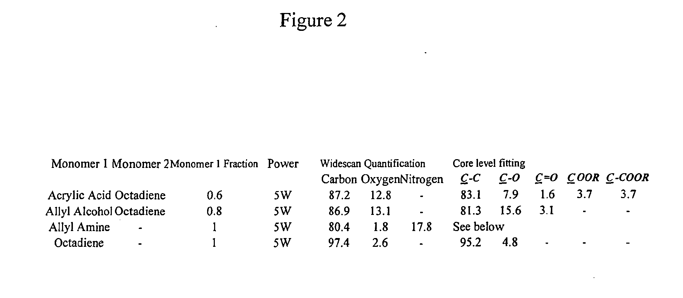

Component peaks representing different chemical environments were fitted to the core-level data using well established binding energy shifts.^{[2]} The results of this curve fitting are shown in Figure 2.

The core level fitting of allyl amine plasma polymers is complicated by the presence of both oxygen and nitrogen functionalities, which in terms of their chemical shifts overlap considerably. By using components peaks representing amine (0.9eV) imine and hydroxyl (1.7eV) and amide (3.0eV) groups, we calculate that the deposit contained 17% amine, 12% imide/hydroxyl and around 2% amide.

### Enzyme Immunoassay

The results of the IgG assay are shown in Figure 2a and 2b each column on the chart representing an average of the optical density taken over eleven microwells and with the twelfth protein-free well subtracted to account for non-specific binding of the primary and secondary reagents to the surfaces.

## Claims

1. An immunoassay product comprising a plasma polymer surface obtainable by plasma polymerisation of a hydrocarbon.

2. An immunoassay product as claimed in claim 1 wherein the plasma polymer is a co-polymer.

3. An immunoassay product as claimed in claim 2 wherein the co-polymer comprises at least one organic monomer and at least one hydrocarbon.

4. An immunoassay product as claimed in claim 3 wherein the monomer comprises a single ethylenically unsaturated organic compound.

5. An immunoassay product as claimed in claim 4 wherein the compound is selected from the group consisting of an alkene, a carboxylic acid, an alcohol and an amine.

6. An immunoassay product as claimed in claim 5 wherein the alkene contains up to 20 carbon atoms

7. An immunoassay product as claimed in claim 6 wherein the alkene contains up to 12 carbon atoms, for example 8 carbon atoms.

8. An immunoassay product as claimed in claim 5 wherein the alcohol is an α-β - unsaturated alcohol.

9. An immunoassay product as claimed in claim 5 wherein the amine is a α-β - unsaturated amine.

10. An immunoassay product as claimed in claim 5 wherein the carboxylic acid is a α-β - unsaturated carboxylic acid.

11. An immunoassay product as claimed in claim 3 wherein the monomer comprises a mixture of two or more ethylenically unsaturated organic compounds.

12. An immunoassay product as claimed in any preceding claim wherein the hydrocarbon is an alkene.

13. An immunoassay product as claimed in claim 12 wherein the alkene is a diene.

14. An immunoassay product as claimed in claim 13 wherein the diene is octa 1,7-diene.

15. An immunoassay product as claimed in any preceding claim wherein the product is a mutiwell assay plate or petridish.

16. An immunoassay product as claimed in claim 15 wherein the assay plate is manufactured from glass or plastic.

17. An immunoassay product as claimed in claim 16 wherein the plastic is polystyrene.

18. An immunoassay product as claimed in any preceding claim for use in the detection of the presence and/or activity of an antibody, or effective part thereof, bound thereto.

19. An immunoassay product as claimed in claim 18 wherein the antibody is a monoclonal antibody, or at least the active Fab fragment thereof.

20. Use of an immunoassay product as claimed in any of claims 1-19 in an immunoassay.

21. Use of an immunoassay product as claimed in any of claims 1 to 19 to assay the presence and/or activity of an antibody, or effective part thereof, bound thereto.

22. A method for performing an immunoassay comprising providing an antibody, or active Fab fragment thereof, or antigen to a substrate wherein the substrate has the characteristics of a surface which has been treated by plasma polymerisation of a hydrocarbon or a co-polymer thereof.
